# EUROPEAN PATENT APPLICATION

(11) **EP 4 786 583 A1**
(43) Date of publication of application: **05.08.2026**
(21) Application number: 23954442.2
(22) Date of filing: 12.10.2023
(51) Int. Cl.: C12N 9/10, C12N 9/14

(54) **METHOD FOR SPECIFICALLY DEGRADING G12V MUTANT PROTEIN OF KRAS AND COMPOSITION THEREFOR**

(30) Priority: 27.09.2023 KR 20230130829
(71) Applicant: Nabigene Inc., Chuncheon-si, Gangwon-do 24341 (KR); KNU-Industry Cooperation Foundation, Chuncheon-si, Gangwon-do 24341 (KR)
(72) Inventor: HWANG, Byung Joon, Chuncheon-si, Gangwon-do 24341 (KR); KEE, Yun, Chuncheon-si, Gangwon-do 24341 (KR); KIM, Gi Seong, Hwaseong-si, Gyeonggi-do 18427 (KR); KIM, Ye Na, Chuncheon-si, Gangwon-do 24322 (KR); LIM, Hoe Eun, Seoul 05667 (KR)
(74) Representative: FRKelly
(86) International application number: PCT/KR2023/015742
(87) International publication number: WO 2025/070859

(57) **Abstract**

The present invention relates to a method for specifically degrading a KRAS mutant (G12V) by treating a cell line expressing the KRAS mutant (G12V) with a complex of a RAS binder binding specifically to the KRAS mutant (G12V) and an E3 ligase NEDD4HECT, and a composition used in the method. When the method developed in the present invention is applied to a cell line expressing a wild-type KRAS, the wild-type KRAS is not degraded. In a KRAS mutant (G12V) cell line, it was confirmed that while a protein level is reduced, ERK activity is inhibited and proliferation and penetration of cancer cells related to the KRAS mutant (G12V) are inhibited

## Description

### [Technical Field]

The present invention relates to a method for specifically degrading a KRAS mutation (G12V) by treating a cell line expressing a KRAS mutation (G12V) with a complex of a RAS binder that specifically binds to a KRAS mutation (G12V) and an E3 ligase NEDD4HECT, and to a composition used in the method.

### [Background Art]

Human cells are composed of various proteins. Numerous proteins perform internal cellular regulatory functions as enzymes that trigger chemical reactions and as signaling molecules that produce hormones; they also regulate these functions by selectively degrading proteins present within the cell. Therefore, the protein ubiquitination system is a mechanism that enables the degradation of specific proteins.

In addition to these functions, protein ubiquitination is involved in a wide range of other activities, including DNA repair through post-translational modification, mRNA export, cell cycle regulation, protein interactions in various signaling mechanisms, protein trafficking, and the removal of ubiquitinated proteins by forming aggresomes that trigger autophagy.

Protein ubiquitination is mediated by ubiquitin. Ubiquitin is a regulatory protein composed of 76 amino acids, and ubiquitination occurs through the sequential action of three enzymes: E1, E2, and E3, which bind to the substrate protein. E1 is an active enzyme that binds to ubiquitin in the presence of ATP, promoting the acyl-adenylation of the ubiquitin C-terminus. Ubiquitin bound to E1 moves to the conjugating enzyme E2 to form a complex, which then binds to the ligase enzyme E3. Subsequently, the amine group of the lysine on the substrate initiates a nucleophilic attack on the glycine at the ubiquitin C-terminus, forming a covalent bond that enables ubiquitin to move from E2 to the substrate protein.

This protein mechanism occurs repeatedly, causing ubiquitin, composed of seven lysine molecules, to bind with other ubiquitins to form a polyubiquitin chain. There are seven types of ubiquitin chains, classified as K6, K11, K27, K29, K33, K48, and K63 depending on which residue is bound. Furthermore, proteins perform various functions depending on the ubiquitin chain bound to them.

### Ubiquitin-Proteasom System (UPS)

Representatively, the degradation of proteins within cells is carried out by mechanisms involving lysosomes and proteasomes. There is a significant difference between these two mechanisms: lysosome-mediated processes lack selectivity or regulatory capabilities, whereas proteasome-mediated processes possess selectivity and regulatory abilities.

Proteasomes are located in the nucleus and cytoplasm and degrade most proteins bound to ubiquitin chains.

Proteasomes are composed of two subunits: the 19S regulatory particle and the 20S core particle. The 19S regulatory particle recognizes proteins bound to ubiquitin chains and induces the degradation of these proteins into peptides via the 20S core particle.

### E3 Ligase

E3 ligases possess specificity, binding to both E2 and substrate proteins to determine which substrate protein is degraded by the proteasome through the process of ubiquitination. Because E3 ligases can confer high levels of specificity and selectivity toward the cell's target substrates, they act as mediators in enzyme cascades. The human proteome encodes over 600 E3s, which can be divided into three classes.

The largest class is RING (Really Interesting New Gene/U-box) type E3, consisting of approximately 600 members; next is HECT (Homologous to E6AP C-Terminus) type E3, composed of about 28 members; and finally, RBR (RING between RING) type E3, composed of about 14 members.

RING E3 ligases act as scaffolds that bring E2 closer to the substrate and as allosteric activators of E2, whereas HECT and RBR E3 ligases catalyze substrate ubiquitination in a two-step reaction. In the first step, activated Ub is accepted from E2 in a thiol exchange reaction for catalytic cysteine, and in the second step, the Ub portion is transferred to the lysine of the target substrate.

Looking at HECT-containing E3 ligases, all HECT E3s at the C-terminus exhibit a catalytic HECT domain consisting of a larger N-terminal lobe containing the E2 binding domain and a C-terminal lobe carrying catalytic cysteine. The two lobes are connected by a flexible hinge region that allows the C-lobe to move to facilitate the transfer of Ub from E2 to E3.

PROTACs, which are drugs currently being developed as a method for the selective removal of target proteins, consist of a ligand-linker-E3 ubiquitin ligase ligand of the target protein. This presents a problem in that they are structurally complex, making it difficult to discover suitable ligand and linker combinations. Furthermore, there is a problem in that the target proteins capable of being degraded by PROTAC are limited, and the degradation efficiency of these target proteins is low. Additionally, there is a concern that the drug may cause toxicity by affecting normal cells as well as the problematic cells.

### [Prior Patent Document]

Republic of Korea Patent Publication No. 10-2017-0096878

### [Disclosure]

### [Technical Problem]

The object of the present invention is to provide a method for the specific degradation of G12V mutants of KRAS protein.

Another object of the present invention is to provide a composition involved in the specific degradation of G12V mutants of KRAS protein.

### [Technical Solution]

To achieve the above object, the present invention provides a method for specifically degrading a G12V mutant protein of KRAS by treating the KRAS G12V mutant protein with a fusion protein of a RAS binder that recognizes kRAS and a HECT(homology to E6-AP carboxyl terminus) domain of NEDD4L (Neural precursor cell expressed developmentally down-regulated 4-like) with the substrate-recognizing region removed.

In the present invention, the RAS binder is preferably a monobody, darpin, or an RAF-ras binding domain, but is not limited thereto.

In one embodiment of the present invention, the RAS binder recognizing the kRAS is preferably one RAS binder selected from the group consisting of iDAb, 12VC1, and K55, but is not limited thereto.

In another embodiment of the present invention, the fusion protein of the RAS binder recognizing kRAS and the HECT domain of NEDD4L from which the substrate-recognizing portion has been removed is preferably one of proteins set forth in SEQ ID NOs 1 to 3, but any mutant that achieves the intended effect of the present invention through one or more substitutions, deletions, inversions, and translocations in the amino acid sequence is also included within the scope of the present invention.

The present invention also provides a composition for degrading a G12V mutant of KRAS, comprising as an active ingredient a fusion protein of the RAS binder recognizing kRAS and the HECT (homology to E6-AP carboxyl terminus) domain of NEDD4L (Neural precursor cell expressed developmentally down-regulated 4-like) with the substrate-recognizing region removed.

In one embodiment of the present invention, the RAS binder recognizing kRAS is preferably, but is not limited to, one RAS binder selected from the group consisting of iDAb, 12VC1, and K55.

In another embodiment of the present invention, the fusion of the RAS binder recognizing kRAS and the HECT domain of NEDD4L from which the substrate-recognizing portion has been removed is preferably one of proteins set forth in SEQ ID NOs 1 to 3, but any mutant that achieves the intended effect of the present invention through one or more substitutions, deletions, inversions, and translocations in the amino acid sequence is also included within the scope of the present invention.

The present invention is described below.

The present invention relates to the specific degradation of the carcinogenic protein KRAS mutation (G12V), and more specifically to a method for specifically degrading the KRAS mutation (G12V) by treating a cell line expressing the KRAS mutation (G12V) with a complex of a RAS binder that specifically binds to the KRAS mutation (G12V) and an E3 ligase NEDD4HECT, and to a composition used in the method. When the method developed in the present invention is applied to a cell line expressing the KRAS wild type, the KRAS wild type is not degraded, and in the KRAS mutation (G12V) cell line, the protein level decreases while ERK activity is inhibited, and it can be confirmed that the proliferation and infiltration of cancer cells associated with the KRAS mutation (G12V) are inhibited.

### [Advantageous Effects]

According to the present invention, when the method developed in the present invention is applied to a cell line expressing KRAS wild type, the KRAS wild type is not degraded, and in KRAS mutant (G12V) cell lines, the protein level decreases and ERK activity is inhibited, and it can be confirmed that the proliferation and infiltration of cancer cells associated with KRAS mutant (G12V) are inhibited.

### [Description of Drawings]

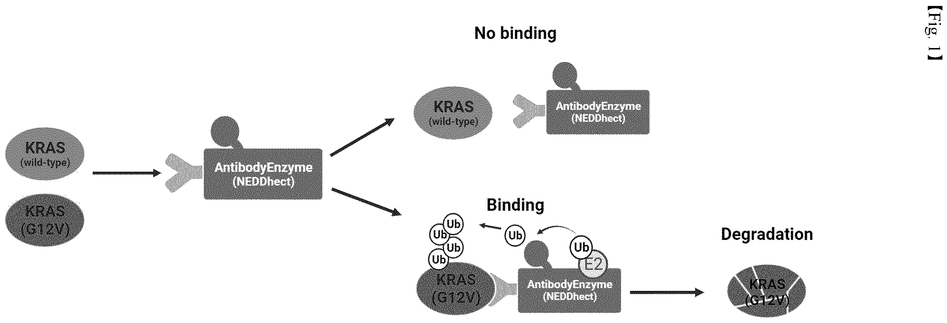

FIG. 1 is a schematic diagram of the present invention,

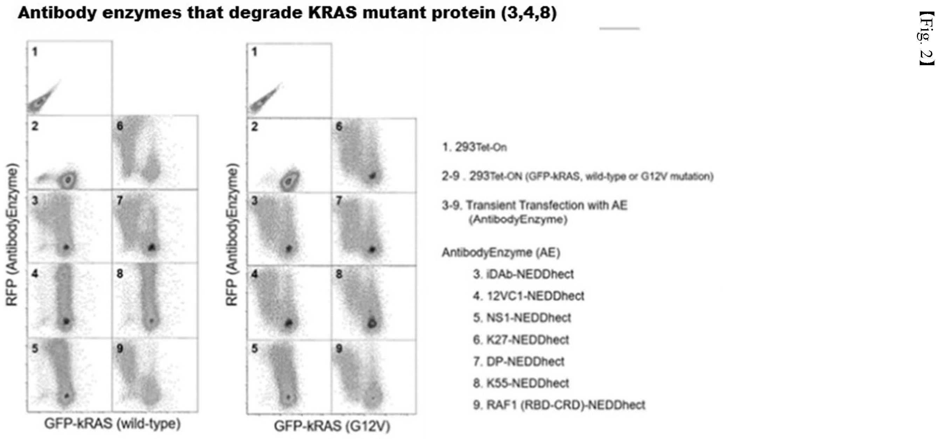

Fig. 2 is a figure showing antibody enzymes that degrade kRAS variant protein, and such effects are observed in numbers 3, 4, and 8.

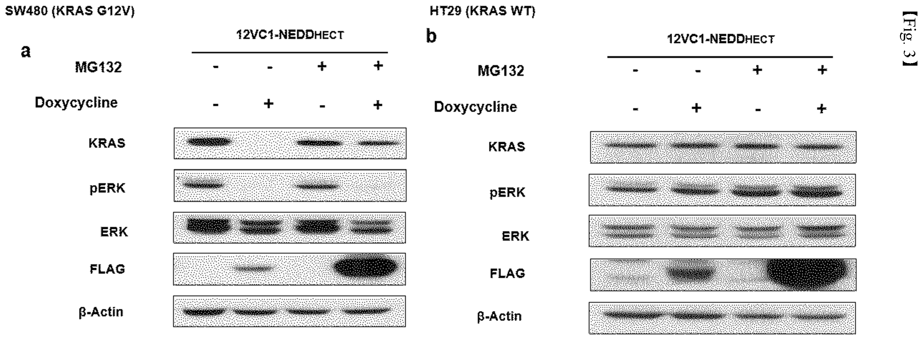

Figure 3(a) shows the degradation of KRAS G12V by 12VC1-NEDDHECT. To determine whether KRAS protein can be degraded using 12VC1, a monobody with high affinity for KRAS G12V and G12C, and NEDDHECT, a HECT E3 ubiquitin ligase, experiments were conducted using a SW480 stable cell line in which 12VC1-NEDDHECT is expressed by TRE. To induce the expression of 12VC1-NEDDHECT in the colorectal cancer cell line SW480 (KRAS G12V), Doxycycline (1 µg/mL) was administered for 20 hours, and Western blot analysis confirmed that when 12VC1-NEDDHECT is expressed by treatment with Doxycycline, KRAS protein is effectively degraded. In addition, we confirmed that pERK, a downstream signal of KRAS, decreases when KRAS protein degradation occurs (Lane 1,2),

In addition, to determine whether the degradation of KRAS G12V by 12VC1-NEDDHECT occurs via UPS, the proteasome inhibitor MG132 (10 mM) was treated for 6 hours followed by treatment with Doxycycline (1 µg/mL) for 20 hours. Western blot analysis revealed that KRAS protein degradation occurred effectively in the MG132 (-) / Doxycycline (+) combination, whereas KRAS degradation was inhibited in the MG132 (+) / Doxycycline (+) combination. This confirmed that the degradation of KRAS protein by 12VC1-NEDDHECT occurs via UPS (Lane 3,4).

Figure 3b shows that to confirm the effects of 12VC1, a monobody selective for KRAS G12V and G12C, and NEDDHECT, a HECT E3 ubiquitin ligase, on KRAS WT cells, experiments were conducted using an HT29 stable cell line in which 12VC1-NEDDHECT is expressed by TRE. To induce 12VC1-NEDDHECT expression in HT29, a KRAS WT colorectal cancer cell line, Doxycycline (1 µg/mL) was administered for 20 hours, and Western blot analysis confirmed that even when 12VC-NEDDHECT was expressed, KRAS protein degradation did not occur in KRAS WT cells.

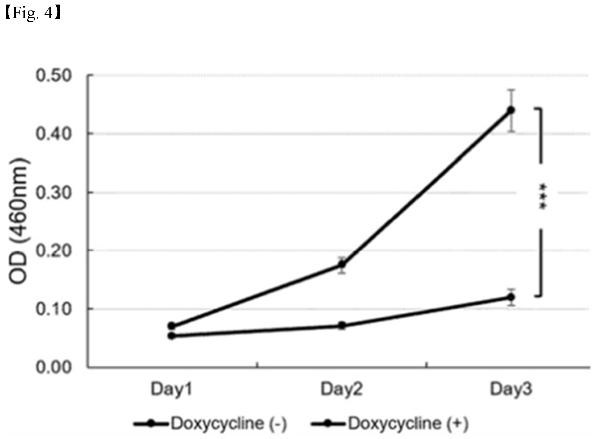

Figure 4 illustrates the effect on target cell proliferation. To determine whether cell growth can be inhibited in SW480, a colorectal cancer cell line with a KRAS mutation, when KRAS (G12V) degradation occurs via 12VC1-NEDDHECT, stable cells (5 x 10³ cells) were prepared and plated in 96-well plates divided into medium containing Doxycycline (1 mg/mL) and medium without Doxycycline. Live cells were identified using CCK-8 (Abcam) after 24, 48, and 72 hours. Absorbance was measured at 460 nm 1 hour and 30 minutes after CCK-8 treatment, and when plotted, it was confirmed that the value after 72 hours significantly decreased in the Doxycycline (+) group. ***p ≤ 0.01

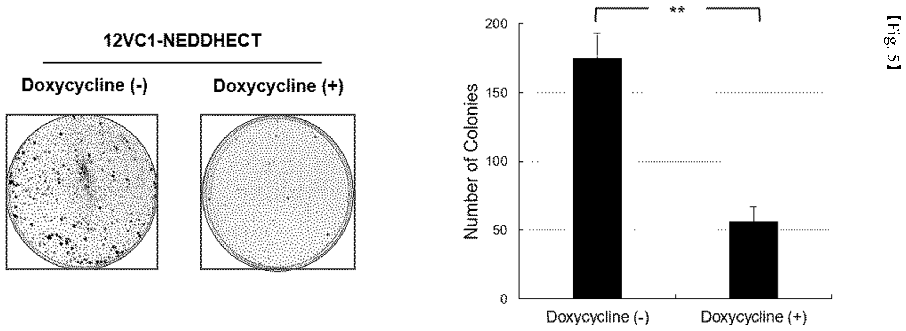

Figure 5 illustrates the effect on target cell growth (colony formation). To determine whether KRAS degradation by 12VC1-NEDDHECT inhibits the colony-forming ability of cells, SW480 Stable cells (2 x 10² cells) were divided into two groups in 6-well plates: one containing Doxycycline (1 mg/mL) and the other not. Doxycycline (1 µg/mL) was added every 72 hours after plating. After 13 days, the colonies were stained with Crystal Violet solution, and the number of colonies was counted to compare colony-forming ability. The results showed a significant decrease in the number of colonies in the group treated with Doxycycline (1 µg/mL). **p < 0.01

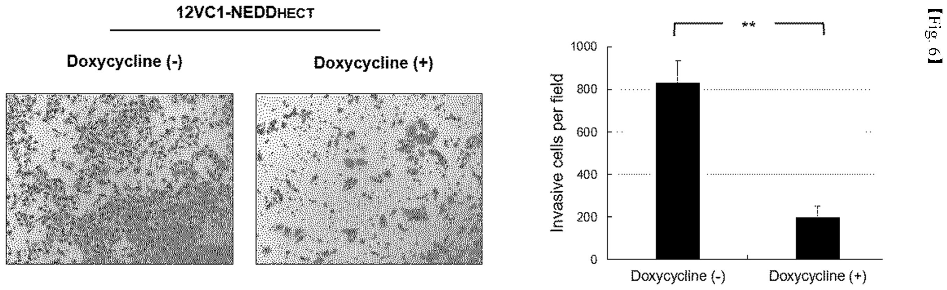

Figure 6 illustrates the effect on target cell invasion. A Matrigel invasion chamber (Corning) was used to determine whether the invasive ability of cells could also be inhibited. Constructed stable cells (1.5 x 10⁵ cells) were added to 8 mm pore Matrigel inserts, divided into negative and positive (1 mg/mL) groups, respectively. After 72 hours, non-invading cells were removed, and cells on the underside of the membrane were fixed and stained with Hematoxylin and Eosin Y. After staining, the number of stained cells was measured under a microscope to count the invaded cells. When the results were plotted on a graph, it was confirmed that the number of invaded cells in the Doxycycline (+) sample was significantly reduced. **p < 0.01,

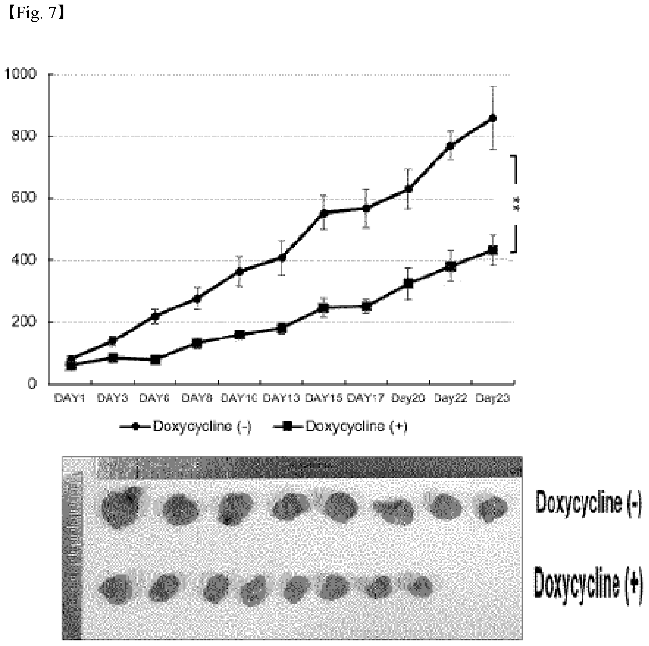

Figure 7 illustrates the in vivo antitumor efficacy in xenograft mice. Nude mice were used to evaluate the in vivo antitumor efficacy of KRAS (G12V) degradation by 12VC1-NEDDHECT. 3 x 10⁶ SW480 Stable cells expressing 12VC1-NEDDHECT via a TRE promoter were inoculated into the left flank of the mice. Eight days after inoculation, mice were randomly assigned to an experimental group or a control group based on tumor volume (approximately 70 mm³). In the experimental group, expression of 12VC1-NEDDHECT was induced by providing Doxycycline (650 mg/kg) via feed. During the experiment, tumor size was measured using a digital caliper, and it was confirmed that the tumor volume in the group in which 12VC1-NEDDHECT expression was induced via Doxycycline was significantly reduced compared to the control group.

### [Mode for Invention]

Hereinafter, the present invention will be described in more detail through examples, but these are merely illustrative and are not intended to limit the scope of the present invention. It is obvious to those skilled in the art that the embodiments described below can be modified without departing from the essential gist of the invention.

### Example 1. Strains and Culture Conditions

### 1) E. coli strain and culture

In the present invention, Escherichia coli Top10F' (F'[lacIq Tn10(tetR)] mcrA Δ(mrr-hsdRMS-mcrBC) φ80lacZΔM15 ΔlacX74 deoR nupG recA1 araD139 Δ(ara-leu)7697 galU galK rpsL(StrR), Invitrogen) was used as the competent cell to be used for amplifying the recombinant plasmid. This E. coli strain was cultured in LB medium (0.5% yeast extract, 1% Tryptone, 0.5% sodium chloride, Lennox) at 37°C and 225 rpm for 12 to 15 hours. To perform drug selection based on the type of plasmid, the antibiotics kanamycin (25 µg/ml) and carbenicillin (100 µg/ml) were added to LB medium and cultured.

### 2) Animal cells and culture

293TetOn cells were cultured in DMEM medium (containing 4.5 g/L glucose, sodium pyruvate, L-glutamine) supplemented with 10% FBS, 1% penicillin/streptomycin, and 10 mM HEPES under conditions of 5% CO2 and 37°C. The 293T-CMV-msfGFP_WT KRAS cells and 293TetOn-CMV-msfGFP_KRAS_G12V cells used in the experiment were cultured (5% CO2, 37°C) in DMEM medium (containing 4.5g/L glucose, L-glutamine, sodium pyruvate, [10% FBS, 10mM HEPES, 1% penicillin/streptomycin]) with puromycin added at a concentration of 2µg/ml to maintain msfGFP_WT KRAS and msfGFP_KRAS_G12V within the cells.

SW480 was cultured under conditions of 5% CO2 and 37°C in RPMI1640 medium (containing 300mg/L L-glutamine) supplemented with 10% FBS, 1% penicillin/streptomycin, and 10mM HEPES. The SW480-Nb139_SPOP167-374 cells used in the experiment were cultured (5% CO2, 37°C) in a medium containing 10% FBS, 1% penicillin/streptomycin, 10mM HEPES, and RPMI1640 (containing 300mg/L L-glutamine) by adding at a concentration of 200µg/ml of hygromycin used to create the cells.

HT-29 was cultured in RPMI1640 medium (containing 300 mg/L L-glutamine) supplemented with 10% FBS, 1% penicillin/streptomycin, and 10 mM HEPES under conditions of 5% CO2 and 37°C. The HT-29-12VC1_NEDDHECT cells used in the experiment were cultured (5% CO2, 37°C) by adding puromycin at a concentration of 40 µg/ml, which was used when creating the cells, to RPMI1640 medium (containing 300 mg/L L-glutamine) supplemented with 10% FBS, 1% penicillin/streptomycin, 10 mM HEPES.

### Example 2. Vector Construction

### 1) Construction of Expression Vector in Animal Cells

In a plasmid containing pEM791-SRa-HygroB_rtTA3, the fluorescent protein (TagRFP) gene and E3 ubiquitin ligase were recombined into a bidirectional TRE (tetracycline response element) promoter. The TRE promoter was used to ensure expression only under conditions where doxycycline is present.

For the experiment requiring analysis using FACS, the fluorescent protein gene RFP670 and TagRFP were amplified via PCR into the TRE promoter and recombined by treatment with restriction enzymes (BstBI /SalI ).

Flags were attached to KRAS-recognizing binders iDAb, 12VC1, NS1, K27, DP KRAS, K55, and RAF1_RBD_CRD, and NEDD4HECT was recombined by treating it with restriction enzymes (XhoI /MluI ). To transform the animal cells used in the experiment, msfGFP fusion proteins (KRAS_WT, KRAS_G12V) synthesized with msfGFP and specific proteins were obtained via PCR, and the resulting PCR product was treated with restriction enzymes and recombined into the pPuro-CMV lentiviral vector.

### 2) Construction of a vector for Lenti virus production

Flag-12VC1-NEDDHECT was amplified by PCR into the TRE (tetracycline response element) promoter of the pCW57.1-DUX4-CA_EcoR I_Nhe I_IRES2_mCherry plasmid and then recombined by treatment with restriction enzymes (Mfe I/Nhe I). The TRE promoter was used to ensure expression only under conditions where doxycycline is present.

### Example 3. Transformation of Recombinant Vectors

### 1) Transformation of E. coli

The mixture containing the vector and the inserted gene, ligated during the cloning process, was mixed with competent cells, Escherichia coli Top10F. After being left on ice for 5 minutes, a heat shock was applied at 42°C for 1 minute to allow the ligated DNA to enter the competent cells. After being left on ice for 3 minutes, LB medium was added, and the cells were cultured at 37°C, 225 rpm, for 1 hour to allow for a recovery period.

Subsequently, the cells were cultured overnight (14-16 hours) in LB agar medium containing the necessary antibiotics.

### 2) Transformation of Animal Cells

Animal cells cultured in a 10 cm plate were treated with 1.5 ml of 0.25% trypsin to harvest the cells. After removing the trypsin by centrifugation, the cells were inoculated into a 6-well plate at a concentration of 7.6 x 10⁵ cells/well. After 24 hours, when the cells had grown to about 60-70%, a mixture of 2 µg DNA, 8 µg Polyethylenimine (PEI), and 300 µl Opti-MEM was added to each well after being left at room temperature for 15 minutes. Then, after incubating for 4 hours under conditions of 5% CO2 and 37°C, 2 µg doxycycline was added.

### Example 4. Fluorescence Analysis Using a Flow Cytometer

### (1) Cell Preparation for Flow Cytometer Analysis

1 ml of 0.25% trypsin was added to each well for 3 minutes, and the cells were collected, placed in 3 ml of complete medium, and centrifuged at 700 rpm for 3 minutes. Afterward, the supernatant was removed, and 1 ml of 1X PBS (Phosphate-buffered saline) was added to suspend the cells. The suspended cells were then placed into BD-Falcon FACS tubes to prepare the samples.

### (2) Flow Cytometer Analysis

The analysis of data obtained through FACS was performed using the FACScaliber^{™} System under GFP (Excitation: 488 / Emission: 509) and TagRFP (Excitation: 555 / Emission: 584) conditions. For GFP, an excitation state was induced using a 488 nm laser, and the emitted wavelength was verified using FL1 (530/30 bandpass filter). TagRFP induced an excitation state using a 488nm laser, and the emitted emission wavelength was verified using FL2 (585/42 bandpass filter). The data was analyzed using the analysis program Kaluza (Backman Coulter).

### Example 5. Cell Line Construction

### (1) Construction of 293T-msfGFP_KRAS_WT, 293Tet-On-msfGFP_KRAS_G12V Cell Lines

To construct the 293T-msfGFP_KRAS_WT, 293Tet-On-msfGFP_KRAS_G12V cell lines, cells were transformed with PEI and selected using a medium containing 2 µg/ml of puromycin.

### (2) Construction of SW480-12VC1-NEDDHECT and HT-29-12VC1-NEDDHECT cell lines using Lentivirus

### (2-1) Lentivirus production

3 x 10⁶ HEK293T cells were seeded in a 100mm dish, and after 24 hours, they were transformed with 12µg of pCW57.1-DUX4-CA_FLAG_12VC1_NEDDHECT_IRES2_mCherry vector, 8.5µg of psPAX2 (lentiviral packaging vector), 2.8µg of pMD2.G (envelope vector), and 68.5µg of PEI. 48 hours after transformation, the supernatant was collected, filtered using a 0.45µm filter, and 3ml of Lenti-X concentrator was added to concentrate the solution for 24 hours. The concentrated mixture was centrifuged at 1,500 x g for 45 minutes at 4°C and then dispersed in 1 ml of medium.

### (2-2) Virus Transduction and Protein Expression

Cells were transduced with lenti virus in 1.5 ml of medium containing 10 µg/ml polybrene in a 6-well plate for 48 hours. Transduced cells were selected with puromycin 40 µg/ml. FLAG_12VC1_NEDDHECT is expressed by the TRE promoter upon treatment with 1 µg/ml of Doxycycline.

### Example 6. Measurement of Target Cell Proliferation

To measure proliferation in target cells, 5 x 10³ cells of the SW480 Stable cell line established were plated in 96-well plates, divided into medium containing Doxycycline (1 µg/mL) and medium without Doxycycline. (Cultured in RPMI1640 medium containing 10% FBS and 1% Penicillin/streptomycin). After 24, 48, and 72 hours, CCK-8 (Abcam) was added to the live cells, and after 1 hour and 30 minutes, the absorbance was measured at 460 nm using a microplate reader.

### Example 7. Measurement of Target Cell Colonies

To measure colony formation in target cells, SW480 Stable cell line 2X102 cells were plated in 6-well plates, divided into medium containing Doxycycline (1 µg/mL) and medium not containing Doxycycline. Doxycycline (1 µg/mL) was added every 72 hours, and after 12-13 days, the cells were washed with PBS, fixed with methanol, stained with Crystal violet solution, and counted.

### Example 8. Measurement of Cell Invasiveness

To measure the invasion ability of target cells, SW480 Stable cell line 1.5 x 10² cells were added to a Matrigel invasion chamber (Corning) insert in separate groups of negative and positive doxycycline (1 mg/mL). After 72 hours, non-invading cells were removed, and cells on the surface beneath the membrane were fixed and stained with Hematoxyclin and Eosin Y.

### Example 9. Xenograft Mouse Experiment

BALB/c-nude mice were used to evaluate in vivo antitumor efficacy. 3 x 10⁶ cells of the SW480 stable cell line (12VC1-NEDDHECT) were inoculated into the left flank of 6-week-old mice. Eight days after inoculation, mice were randomly assigned to an experimental group or a control group based on tumor volume (approximately 70 mm³). In the experimental group, expression of 12VC1-NEDDHECT was induced by providing Doxycycline (650 mg/kg) via feed. During the experiment, tumor size was measured using a digital caliper and plotted on a graph.

## Claims

1. A method for specifically degrading a G12V mutant protein of KRAS by treating the KRAS G12V mutant protein with a fusion protein of a RAS binder that recognizes kRAS and a HECT(homology to E6-AP carboxyl terminus) domain of NEDD4L (Neural precursor cell expressed developmentally down-regulated 4-like) with the substrate-recognizing region removed.

2. The method according to claim 1, wherein the RAS binder recognizing the kRAS is one RAS binder selected from the group consisting of iDAb, 12VC1, and K55.

3. The method according to claim 1, wherein the fusion protein of the RAS binder recognizing kRAS and the HECT domain of NEDD4L with the substrate-recognizing region removed is one of proteins set forth in SEQ ID NOs 1 to 3.

4. A composition for degrading a G12V mutant of KRAS, comprising a fusion protein of the RAS binder recognizing kRAS and the HECT (homology to E6-AP carboxyl terminus) domain of NEDD4L (Neural precursor cell expressed developmentally down-regulated 4-like) with the substrate-recognizing region removed.

5. The composition according to claim 4, wherein the RAS binder recognizing the kRAS is one RAS binder selected from the group consisting of iDAb, 12VC1, and K55.

6. The composition according to claim 4, wherein the fusion protein of the RAS binder recognizing kRAS and the HECT domain of NEDD4L with the substrate-recognizing region removed is one of proteins set forth in SEQ ID NOs 1 to 3.
